Europäisches Patentamt
European Patent Office
Office européen des brevets

(19)

(11) Publication number: **0 094 739**
**A1**

(12) **EUROPEAN PATENT APPLICATION**

(21) Application number: **83301855.9**

(22) Date of filing: **31.03.83**

(51) Int. Cl.[3]: **C 07 C 107/02**
**C 08 F 4/04**

(30) Priority: **13.05.82 GB 8213880**

(43) Date of publication of application:
**23.11.83 Bulletin 83/47**

(84) Designated Contracting States:
**BE DE FR GB IT NL**

(71) Applicant: **IMPERIAL CHEMICAL INDUSTRIES PLC**
**Imperial Chemical House Millbank**
**London SW1P 3JF(GB)**

(72) Inventor: **Davies, Stephen Parry**
**26 Clara Street South Yarra**
**Melbourne Victoria 3141(AU)**

(72) Inventor: **Thompson, Morice William**
**Urishay Highfield Lane**
**Cox Green Maidenhead Berkshire(GB)**

(74) Representative: **Wood, Dennis John Cecil et al,**
**Imperial Chemical Industries PLC Legal Department:**
**Patents Thames House North Millbank**
**London SW1P 4QG(GB)**

(54) **Novel azocarboxylic esters useful as polymerisation initiators and process for their manufacture.**

(57) Azocarboxylic esters useful as polymerisation initiators, especially for polymerisation in aqueous medium, have the general formula

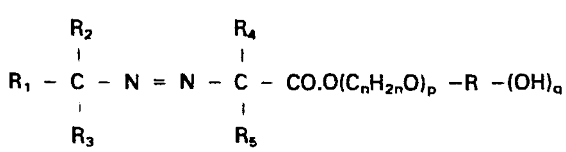

in which R is the residue of an aliphatic polyhydroxy compound $R(OH)_{q+1}$ after removal of one hydroxyl group therefrom, $R_1$ is a methyl or ethyl group or a grouping of the formula $-COO(C_nH_{2n}O)_p-R(OH)_q$; $R_2$ is a methyl group or a βcarboxyethoxy group and $R_3$ is a methyl group or an ethyl group, or $R_2$ and $R_3$ together with the intermediate carbon atom form a cyclohexyl group; $R_4$ is a methyl group and $R_5$ is a methyl group, an ethyl group, a β-carboxyethoxy group or a β-methoxy-ββ-dimethylethyl group, or $R_4$ and $R_5$ together with the intermediate carbon atom form a cyclohexyl group; n has one or more of the values 2, 3 and 4, p is zero or an integer from 1 to 100, preferably from 1 to 50, and q has the value 2 or 4. The esters can be prepared by reacting a corresponding azonitrile or azobisnitrile with a stoichiometrically equivalent amount of an aceral or ketal of the formula

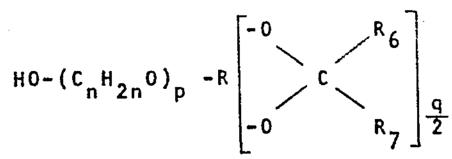

wherein $R_6$ is hydrogen or an alkyl or aryl group and $R_7$ is an alkyl or artl group, or $R_6$ and $R_7$ together with the intermediate carbon atom form a cyclohexyl group, in the presence of hydrogen chloride gas under anhydrous conditiond, followed by hydrolysis of the product.

EP 0 094 739 A1

Croydon Printing Company Ltd

# NOVEL AZOCARBOXYLIC ESTERS USEFUL AS POLYMERISATION INITIATORS AND PROCESS FOR THEIR MANUFACTURE

This invention relates to novel organic azo compounds which are useful as initiators for the polymerisation of ethylenically unsaturated monomers, and to a process for their manufacture.

The use of organic azo compounds as initiators in the aqueous emulsion polymerisation of ethylenically unsaturated monomers is well known and extensively described in the literature. Illustrations of such compounds are azobis(2-methylpropionitrile), 4,4'-azobis-(4-cyanopentanoic acid), 2,2'-azobis(2-cyanoethyl-p-benzoic acid), 4,4'-azobis(4-cyano-n-pentanol), 2,2'-azobis(4-amino-2, 4-dimethylvaleronitrile) and 1,1'-azobis(1-cyanocyclohexane).

According to one aspect of the present invention there are provided azo carboxylic esters having the general formula:-

$$R_1 - \overset{\displaystyle R_2}{\underset{\displaystyle R_3}{C}} - N = N - \overset{\displaystyle R_4}{\underset{\displaystyle R_5}{C}} - CO.O(C_nH_{2n}\ O)_p - R\ (OH)_q \qquad (I)$$

in which R is the residue of an aliphatic polyhydroxy-compound $R(OH)_{q+1}$ after removal of one hydroxyl group therefrom, $R_1$ is a methyl or ethyl group or a grouping of theformula $-COO(C_nH_{2n}O)_p\ -R(OH)_q$ ; $R_2$ is a methyl group or a $\beta$-carboxyethoxygroup and $R_3$ is a methyl group or an ethyl group, or $R_2$ and $R_3$ together with the intermediate carbon atom form a cyclohexyl group; $R_4$ is a methyl group

and $R_5$ is a methyl group, an ethyl group, a β-carboxyethoxy group or a β-methoxy-ββ-dimethylethyl group, or $R_4$ and $R_5$ together with the intermediate carbon atom form a cyclohexyl group; n has one or more of the values 2, 3 and 4, p is zero or an integer from 1 to 100, preferably from 1 to 50, and q has the value 2 or 4.

According to another aspect of the present invention there is provided a process for the manufacture of azo carboxylic esters as defined in formula (I), the process comprising reacting an azonitrile of the formula

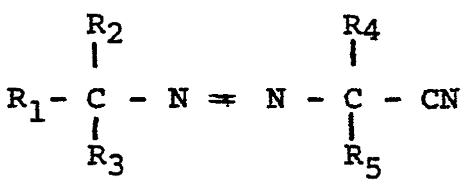

(II)

wherein $R_1$ is a methyl group, an ethyl group or a nitrile group, and $R_2$, $R_3$, $R_4$ and $R_5$ have the same significance as in formula (I), with a stoichiometrically equivalent amount of an acetal or ketal derivative having the formula

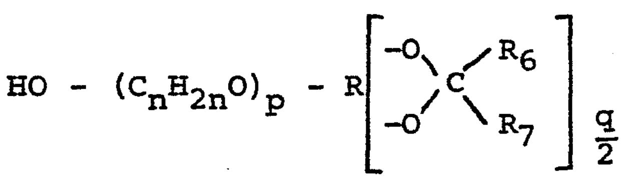

(III)

wherein R, n, p and q have the same significance as in formula (I), $R_6$ is hydrogen or an alkyl or aryl group and $R_7$ is an alkyl or aryl group, or $R_6$ and $R_7$ together with the intermediate carbon atom form a cycloalkyl group, in the presence of hydrogen chloride gas under anhydrous conditions, followed by simultaneous hydrolysis of the intermediate iminoether hydrochloride and of the acetal or ketal grouping or groupings.

The azo moiety of the compounds (I) may be derived from such azonitriles (II) as 2,2'-azobis(2-methylpropionitrile), 2,2'-azobis(2-methylbutyronitrile), 4,4'-azobis(4-cyanopentanoic acid), 1,1'-azobis(1-cyanocyclohexane), 2-(2'-methylpropan-2'-azo)-2-cyano-4-methoxy-4-methylpentane, 1-(2'-methylpropan-2'-azo)-1-cyanocyclohexane), 1-(2'-methylbutan-2'-azo)-1-cyanocyclohexane, 2-(2'-methylpropan-2'-azo)-2-cyanopropane and 2-(2'-methylpropan-2'-azo)-2-cyanobutane.

The ester moiety of the compounds (I) may be derived from polyhydroxy compounds (III) having 3 or 5 hydroxyl groups, or from a poly(alkyleneoxy) derivative of such a compound in which only one of the hydroxyl groups is substituted by a polyether chain. The poly(alkyleneoxy) chain may consist entirely of ethyleneoxy units, or of propyleneoxy units, or of butyleneoxy units, or it may include units of any two or all three of these types. Furthermore, in the latter case, the different units may be arranged within the chain either randomly or in blocks. Examples of suitable polyhydroxy compounds include glycerol, trimethylolpropane, 1,2,6-hexanetriol and carbohydrates such as D-glucofuranose together with their condensates with alkylene oxides such as ethylene oxide, propylene oxide and butylene oxide or mixtures thereof, provided that, as already indicated, in the case of the condensates the alkylene oxide units all form a single chain attached to the site of only one of the hydroxyl groups of the polyhydroxy compound.

The azocompounds of the invention may be obtained from the corresponding azonitriles by means of the modified Pinner reaction defined above. The monohydroxy compound with which the azonitrile is reacted is an acetal or ketal derivative of the polyhydroxy compound $R(OH)_{q+1}$ in which one pair, or two pairs as the case may be, of hydroxyl groups are "blocked" by being incorporated into

1:3-dioxolan or 1:3-dioxan ring systems. The acetal or ketal derivatives are themselves well known substances which are produced by condensing the polyhydroxy compound, under anhydrous conditions in the presence of a catalyst, with the appropriate aldehyde or ketone, such as acetone, methyl ethyl ketone, cyclohexanone, benzaldehyde or acetophenone. Suitable derivatives include 2,2-dimethyl-1, 3-dioxolan-4-methanol (1:2-O-isopropylidene glycerol) and its condensate with 4, 6, 8 or 30 moles of ethylene oxide respectively, the corresponding 2-phenyl-, 2-phenyl-2-methyl-, 2-methyl-2-ethyl- and 2,2-spiro-cyclohexyl- derivatives of 1,3-dioxolan-4-methanol, together with the ethylene oxide condensates thereof, also 1,2:5,6-di-O-isopropylidene glucofuranose and its ethylene oxide condensates. Where it is desired to use the alkylene oxide condensates, these will normally be obtained by preparing the acetal or ketal derivative first and then condensing this with the alkylene oxide(s) in the presence of an alkaline catalyst, towards which the dioxolan or dioxan ring structure is stable.

According to the conventional procedure for carrying out the Pinner reaction, as originally described in Chem.Ber. 1883, 16, 352 and 1643, a nitrile is converted to the corresponding iminoether hydrochloride by treatment with an alcohol in the presence of hydrogen chloride gas under anhydrous conditions, the alcohol being used in stoichiometric excess so as to ensure complete conversion of the nitrile. In suitable cases, particularly when the lower aliphatic alcohols are used, the iminoether hydrochloride precipitates out from the reaction mixture and can be separated from unreacted alcohol, following which it may be hydrolysed to give the corresponding carboxylic

ester. However, in many other instances, the iminoether salt does not precipitate out and its isolation can then be very troublesome. The need to use an excess of the hydroxy compound is also a disadvantage when that compound is expensive or notfreely available. In our European Application No. 82305791.4 there is described a modification of the Pinner procedure according to which there is used only a stoichiometric proportion of hydroxy compound to nitrile and the reaction is carried out in the presence of a compound containing an ether grouping. In a particular embodiment of this modified procedure, the hydroxy compound itself contains the ether grouping, and this same feature is utilised in the process of the present invention, where the dioxolan or dioxan ring in the compound (III) serves to provide the necessary ether groupings.

As described in the above-mentioned Application, the reaction between the azonitrile (I) and the monohydroxy compound (III) is most conveniently brought about by bubbling dry hydrogen chloride gas through the mixture of these reagents until the mixture is saturated, the temperature of the mixture preferably being held below about 10°C by suitable cooling. When saturation has been achieved, the reaction mixture may be allowed to warm up to room temperature, at which the conversion of the nitrile to the iminoether hydrochloride normally occurs satisfactorily over a period of some days. It is not usually desirable to carry out the conversion at temperatures much above ambient since the iminoether salt formed may then decompose to form the corresponding amide. If a faster conversion is desired,this may be achieved by increasing the concentration of hydrogen chloride in the reaction mixture by introducing it under pressure.

If desired, the reaction mixture may also contain a liquid diluent or solvent which is inert towards any of the other reactants and which preferably is sufficiently volatile to be removed, after the reaction is complete, by evaporation at a relatively low temperature (if necessary under reduced pressure) in order not to cause decomposition of the iminoether salt. Particularly suitable diluents include dichloromethane and chloroform; benzene and toluene may also be used,for example where complete removal of the diluent from the iminoether salt is not necessary.

Completion of the conversion of the nitrile to the iminoether hydrochloride may most conveniently be recognised by the absence of the characteristic nitrile group peak at 2250 $cm^{-1}$, on infrared spectroscopic examination of the reaction mixture. The iminoether hydrochloride, in those cases where it separates as a solid from the reaction mixture, may be separated by filtration and, where it does separate, may be isolated by removing the liquid diluent and hydrogen chloride, preferably at about room temperature and, if necessary, under reduced pressure as already mentioned. Further purification of the iminoether salt may be carried out if required, but this is often a matter of some difficulty owing to the limited stability of this class of compound, and normally it is unnecessary since the compound in question is required only as an intermediate product.

Hydrolysis of the iminoether hydrochloride to the carboxylic ester may be accomplished simply by adding the hydrochloride to water at room temperature, and simultaneously there takes place hydrolysis of the acetal or ketal groupings in the ester moiety, giving rise to

free hydroxyl groups. The desired product is in most cases best isolated by extraction into an inert solvent, such as dichloromethane, followed by evaporation of the solvent under reduced pressure.

The resulting azocarboxylic esters (I) are valuable as free radical-forming initiators for the polymerisation of ethylenically unsaturated monomers. By virtue of the free hydroxyl groups which they contain, they are in general readily soluble in water and are therefore particularly suitable for use in aqueous emulsion polymerisation procedures, or in the aqueous dispersion polymerisation processes described in British Application No. 2039497A.

The invention is illustrated but not limited by the following Examples, in which parts are by weight.

## EXAMPLE 1

2,2-Dimethyl-1,3-dioxolan-4-methanol(1:2-O-isopropylidene-glycerol) (264 g, 2 mols) was dried by azeotropic distillation with cyclohexane. 2,2'-Azobis(2-methylpropionitrile) (20 g, 0.12 mol) was added and dry hydrogen chloride gas was passed through the mixture with cooling and stirring, keeping the temperature below about 10°C, until it was saturated. After it had stood at room temperature for 5 days, the hydrogen chloride was partly removed by distillation under reduced pressure and aqueous ammonia was then added so as to bring the pH to about 8. The resulting aqueous solution was extracted with dichloromethane (400 ml). The extract was washed with aqueous ammonia, dried over anhydrous magnesium sulphate and concentrated under reduced pressure. The residue (about 83g),

on infra-red spectroscopic examination, showed a large ester peak at 1735 $cm^{-1}$ but no nitrile absorption. The product was concluded to be substantially the 1,2-dihydroxypropyl ester of 2,2'-azobis(2-methylpropanoic acid) having the formula:-

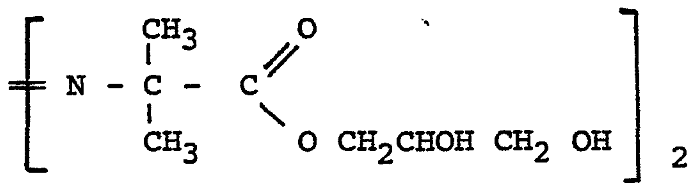

EXAMPLE 2

The reaction product of 2,2-dimethyl-1,3-dioxolan-4-methanol and four molar equivalents of ethylene oxide (154g, 0.5 moles) was dissolved in dichloromethane (350 ml) and 2,2'-azobis(2-methylpropionitrile)(41 g, 0.25 moles) was added.

Dry hydrogen chloride gas was passed through the stirred solution until it was saturated, keeping the temperature of the mixture below 10°C. After two days at room temperature, infra-red spectroscopic examination of the mixture indicated almost complete abesnce of the characteristic $-C\equiv N$ bond at 2250 $cm^{-1}$ , and solvents were then removed under reduced pressure below 10°C. The resulting residue was a mobile, orange-coloured liquid, which was poured into aqueous sodium bicarbonate and then extracted into dichloromethane (3 x 200 ml). The organic extracts were combined, washed with water (3 x 200 ml) and then dried with anhydrous magnesium sulphate. The solvent was removed under reduced pressure and the product was obtained as a yellow liquid. (114.9 g, 59% yield). Infra-red examination showed inter alia a strong broad band at 3,500 $cm^{-1}$ (OH) and a strong band at 1735 $cm^{-1}$ (-ester).

The product was concluded to be substantially the ester of 2,2'-azobis(2-methylpropanoic acid) and 1- [tetra(ethyleneoxy)]-glycerol, having the formula:-

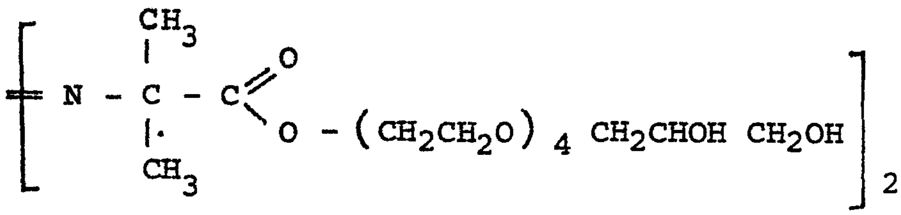

EXAMPLE 3

The reaction product of 2,2-dimethyl-1, 3-dioxolan-4-methanol and 30 molar equivalents of ethylene oxide (144 g, 0.1 moles) was dissolved in dichloromethane (500 mls) and 1-(2'-methylpropan-2'-azo)-1-cyanocyclohexane (19.4 g, 0.1 mole) was added. The solution was stirred and cooled and dry hydrogen chloride was bubbled through it until no more gas was dissolved. After 5 days at room temperature, infra-red examination of the mixture indicated the virtual absence of any nitrile band at 2250cm$^{-1}$. Solvents were removed under reduced pressure the residue poured into saturated aqueous sodium bicarbonate solution (500 mls) and then extracted into dichloromethane (4 x 200 mls). The extract was washed with water (4 x 200 mls), dried (anhydrous magnesium sulphate) and concentrated under reduced pressure to give a viscous oil (81.4 g). Infra-red examination of this product showed a strong broad band at 3600 cm$^{-1}$ (OH), a band at 1735 cm$^{-1}$ (ester) and a band at 1650 cm$^{-1}$ (corresponding to the imino-ether hydrochloride grouping). The product was concluded to be substantially the ester of 1-(2'-methylpropan-2'-azo)-1-cyclohexanecarboxylic acid with 1-[poly(ethyleneoxy)]-glycerol having the formula:-

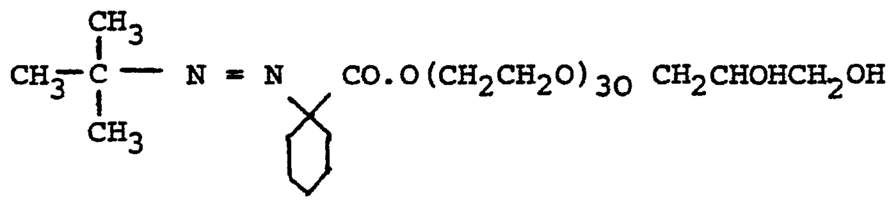

but to contain a proportion of the unhydrolysed intermediate iminoether hydrochloride.

EXAMPLE 4

2,2-Dimethyl-1, 3-dioxolan-4-butanol (48 g, 0.276 mol) was dried by azeotropic distillation with toluene and 2,2'-azobis(2-methylpropionitrile)(5.65 g, 0.034 mol) was added together with dichloromethane (about 100 ml). Dry hydrogen chloride gas was passed through the mixture with stirring and cooling, keeping the temperature below about 10°C, until it was saturated. The deep cherry-red mixture was allowed to stand for 7 days at room temperature, after which the hydrogen chloride and dichloromethane were removed under reduced pressure. Dichloromethane (200 ml) and water (100 ml) were added and the mixture shaken. The organic layer was separated, dried with anhydrous magnesium sulphate and concentrated under reduced pressure to yield a dark brown liquid (15.8g). Infra-red spectroscopic examination of this product showed the absence of any nitrile absorption but the presence of a strong band at 1710 $cm^{-1}$ (ester group) together with bands at 3400 $cm^{-1}$ and at 1370 $cm^{-1}$ (hydroxyl groups).

The product, which was soluble in water but insoluble in methyl methacrylate, was concluded to be substantially the 5:6-dihydroxyhexyl ester of 2,2'-azobis-(2-methy-propanoic acid), having the formula:-

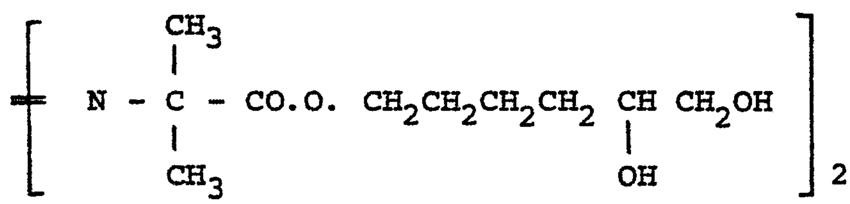

## EXAMPLE 5

The condensate of 1 mol of 1,2 : 5,6-di-O-isopropylidene-D-glucofuranose with 24 mols of ethylene oxide (131.7 g, 0.1 mole) was dissolved in dry dichloromethane (500 ml) and 2,2'-azobis(2-methylpropionitrile) (8.2 g, 0.05 mole) was added. The resulting solution was cooled in an ice-bath and stirred whilst dry hydrogen chloride gas was bubbled into it. After 6 hours, the solution was saturated. The reaction mixture was then allowed to stand at 20°C for 1 week, after which time infra-red spectroscopic examination showed the absence of nitrile groups. The product was isolated in the manner described in Example 4, giving a straw-coloured, waxy solid (82.5 g, 65% yield) which showed a strong ester carbonyl absorption band at 1735 $cm^{-1}$, a very strong C - O - C band at 1100 $cm^{-1}$ and a hydroxyl band at 3500 $cm^{-1}$. The product was concluded to be substantially the ester of 2,2'-azobis-(2-methyl-propanoic acid) with 4-[poly(ethyleneoxy)] -D-glucofuranose having the formula:-

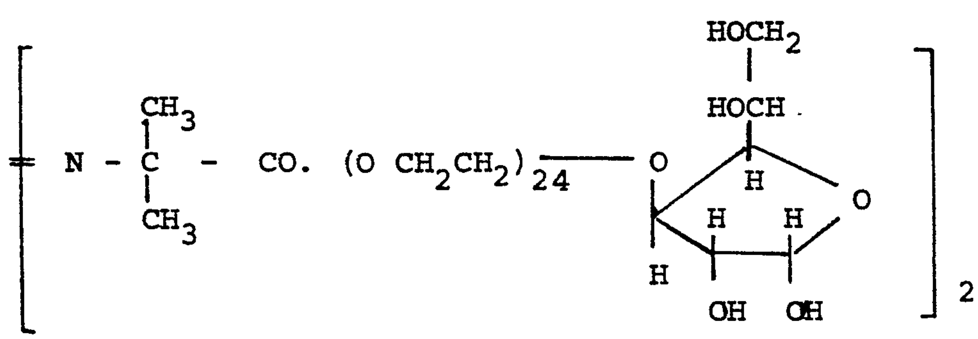

0094739
PV. 32305

WE CLAIM :-

1. Azocarboxylic esters having the general formula :-

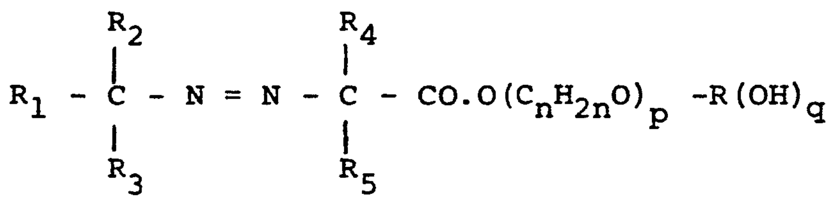

(I)

in which R is the residue of an aliphatic polyhydroxy-compound $R(OH)_{q+1}$ after removal of one hydroxyl group therefrom, $R_1$ is a methyl or ethyl group or a grouping of the formula $-COO(C_nH_{2n}O)_p -R(OH)_q$ ; $R_2$ is a methyl group or a β-carboxyethoxy group and $R_3$ is a methyl group or an ethyl group, or $R_2$ and $R_3$ together with the intermediate carbon atom form a cyclohexyl group; $R_4$ is a methyl group and $R_5$ is a methyl group, an ethyl group, a β-carboxyethoxy group or a β-methoxy-ββ-dimethylethyl group, or $R_4$ and $R_5$ together with the intermediate carbon atom form a cyclohexyl group; n has one or more of the values 2, 3 and 4, p is zero or an integer from 1 to 100, preferably from 1 to 50, and q has the value 2 or 4.

2. Esters as claimed in claim 1, wherein the azo moiety is derived from 2,2'-azobis(2-methylpropionitrile) or 1-(2'-methylpropan-2'-azo)-1-cyanocyclohexane.

3. Esters as claimed in claim 1, wherein the ester moiety is derived from glycerol, trimethylolpropane, 1,2,6-hexanetriol or D-glucofuranose or the alkylene oxide condensates thereof.

4. The azocarboxylic ester having the formula :-

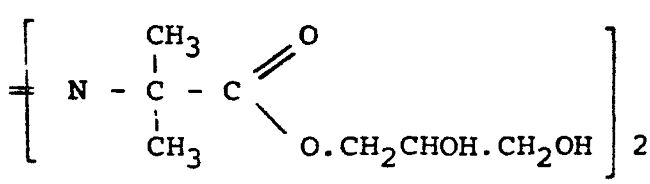

5. The azocarboxylic ester having the formula:-

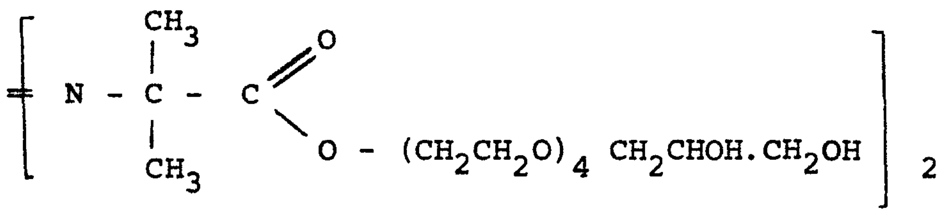

6. The azocarboxylic ester having the formula:-

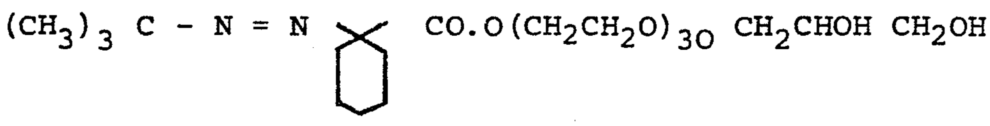

7. The azocarboxylic ester having the formula:-

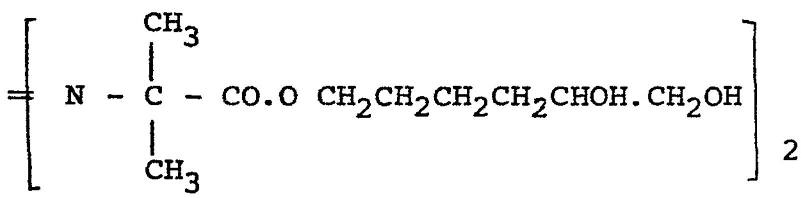

8. The azocarboxylic ester having the formula:-

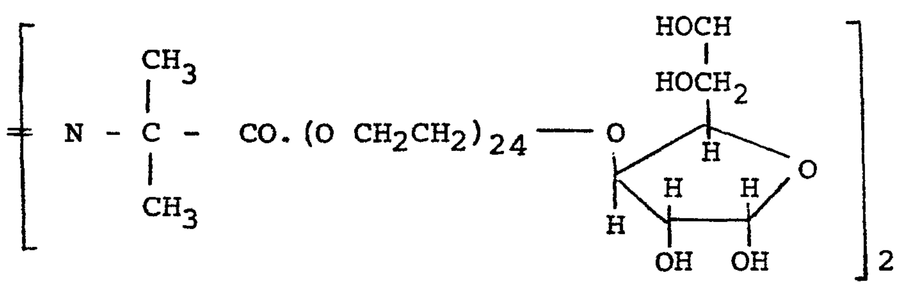

9. A process for the manufacture of azocarboxylic esters as claimed in claim 1, comprising reacting an azonitrile of the formula :-

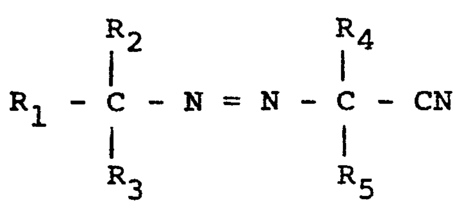

(II)

wherein $R_1$ is a methyl group, an ethyl group or a nitrile group, and $R_2$, $R_3$, $R_4$ and $R_5$ have the same significance as in formula (I), with a stoichiometrically equivalent amount of an acetal or ketal derivative

having the formula :-

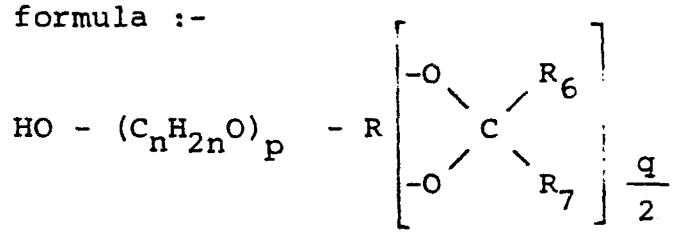 (III)

wherein R, n, p and q have the same significance as in formula (I), $R_6$ is hydrogen or an alkyl or aryl group and $R_7$ is an alkyl or aryl group, or $R_6$ and $R_7$ together with the intermediate carbon atom form a cycloalkyl group, in the presence of hydrogen chloride gas under anhydrous conditions, followed by simultaneous hydrolysis of the intermediate iminoether hydrochloride and of the acetal or ketal grouping or groupings.

0094739

European Patent Office

# EUROPEAN SEARCH REPORT

Application number

EP 83 30 1855

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | DE-A-2 124 000 (MERCK)<br>* Page 1, formula 1; page 4, formula 4 *<br>--- | 1 | C 07 C 107/02<br>C 08 F 4/04 |
| Y | H.M. FLOWERS: "The chemistry of the hydroxyl group", part 2, chapter 18, pages 1002-1003, 1028-1035, 1043-1044, edited by S. Patai, 1971, Interscience Publishers, London, G.B.<br>* Page 1030, lines 17-20 *<br>--- | 1 | |
| A | FR-A-2 384 801 (OLIN)<br>* Page 4, formula 1; page 8, lines 22-29 *<br>----- | 1 | |
| | | | TECHNICAL FIELDS SEARCHED (Int. Cl. 3) |
| | | | C 07 C<br>C 08 F |

The present search report has been drawn up for all claims

| Place of search | Date of completion of the search | Examiner |
|---|---|---|
| THE HAGUE | 22-08-1983 | DAUKSCH H.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82